# DEMANDE DE BREVET EUROPEEN

(11) **EP 1 118 657 A1**
(43) Date de publication de la demande: **25.07.2001**
(21) Numéro de dépôt: 01400108.5
(22) Date de dépôt: 15.01.2001
(51) Int. Cl.: C12M 1/00, B65D 88/62

(54) **Dispositif de culture cellulaire et tissulaire à circulation perfectionnée de fluide de culture.**

(30) Priorité: 17.01.2000 FR 0000548
(71) Demandeur: Cell Tissue Progress, 75017 Paris (FR)
(72) Inventeur: Sarem, Farzin, L'Ile verte 06560 Valbonne (FR); Sarem Damerdji, Leila-Oussila, L'Ile verte 06560 Valbonne (FR)
(74) Mandataire: Vaillant, Jeanne

(57) **Abrégé**

Un dispositif de culture de cellules et tissus comprend plusieurs puits de culture (18-i) logeant des cellules ou des tissus à cultiver, de premier (2) et second (25) réservoirs, et des moyens de liaison (20,23,24) couplés aux puits et aux réservoirs pour permettre la circulation d'un fluide de culture d'un réservoir à l'autre via les puits. Chaque réservoir (20,25) loge au moins une poche souple (6,7 ;27,29), dont l'une au moins est propre à recevoir le fluide de culture. Le dispositif comprend en outre au moins un module de commande (50,150) fournissant de première et/ou de seconde séquences de pressions externes à appliquer, respectivement, sur les poches des premier et seconds réservoirs, et des moyens de pressurisation (41,46-49) agencés pour appliquer aux poches les pressions définies par lesdites première et seconde séquences fournies par le module de commande.

## Description

L'invention concerne le domaine de la culture de cellules et de tissus à l'aide d'un fluide de culture, ou milieu nutritif.

Elle concerne plus précisément les dispositifs et procédés de culture de cellules et tissus dans lesquels le fluide de culture (ou nutriment) est mis en mouvement de manière à permettre une culture dynamique. Ces dispositifs mettent en oeuvre généralement une technique d'agitation directe du fluide de culture ou une technique de circulation permanente de ce fluide.

La présente invention vise plus spécifiquement les dispositifs à circulation de fluide, qui comprennent au moins un puits de culture définissant une chambre dans laquelle sont logés les cellules ou tissus à cultiver, des premier et second réservoirs, dont l'un au moins est agencé de manière à recevoir un fluide de culture, ainsi que des moyens de liaison couplant le puits aux premier et second réservoirs. Le fluide de culture circule d'un réservoir à l'autre via le puits.

Cette technique de circulation de fluide est difficile à maîtriser du fait que les conditions de culture évoluent au cours du temps. En effet, au début de la culture, la faible concentration cellulaire requiert un environnement quasi-statique, et par conséquent un débit de fluide de culture très faible. Puis le débit croît rapidement en raison de la multiplication des cellules ou de la croissance des tissus.

Le maintien des conditions adéquates pendant toute la durée de la culture requiert soit l'utilisation de plusieurs dispositifs appropriés respectivement aux différents stades d'évolution de la culture, ce qui impose de nombreuses manipulations, soit des moyens complexes et souvent encombrants, qui limitent, voire interdisent, l'observation de l'évolution de la culture à l'aide d'un microscope. Ces manipulations et moyens sont d'autant plus complexes que la culture doit s'effectuer dans un environnement stérile qui requiert du personnel hautement qualifié, et par conséquent accroît le coût de chaque culture. De plus, du fait de leur coût, ces dispositifs doivent être réutilisables, ce qui impose une stérilisation complète avant chaque nouvelle culture et des opérations de maintenance .

L'invention a pour but de résoudre tout ou partie des inconvénients précités.

Elle propose à cet effet un dispositif du type décrit dans l'introduction, dans lequel chacun des premier et second réservoirs loge au moins une poche souple, l'une au moins des poches de ces réservoirs étant adaptée pour recevoir le fluide de culture, et qui comprend au moins un module de commande permettant de fournir de première(s) et/ou seconde(s) séquences de pressions externes à appliquer, respectivement, sur les poches des premier et seconds réservoirs, et des moyens de pressurisation agencés pour appliquer aux poches les pressions définies par les première et seconde séquences fournies par le module de commande.

Ainsi, il suffit de définir pour chaque type de culture des séquences de pression à appliquer sur les différentes poches, puis de communiquer au module de commande ces séquences pour que la culture s'effectue pendant toute sa durée dans des conditions adéquates. Cette communication peut s'effectuer indirectement à l'aide d'une interface de saisie, ou bien directement à l'aide d'une mémoire dans laquelle se trouve préalablement stockées une multiplicité de séquences en correspondance d'échantillons différents (cette mémoire étant éventuellement réinscriptible par l'intermédiaire de l'interface précitée).

Dans un mode de réalisation préférentiel du dispositif selon l'invention, les premier et second réservoirs comprennent chacun une partie supérieure et une partie inférieure qui sont reliées entre elles par une partie intermédiaire étroite et qui logent chacune une poche souple, les poches souples supérieure et inférieure communiquant entre elles via la partie intermédiaire, et les moyens de liaison communiquant avec les poches inférieures.

Dans ce mode de réalisation, il est particulièrement avantageux que le module de commande puisse fournir des séquences de pressions propres à chaque poche, de sorte que les moyens de pressurisation appliquent sur chacune de ces poches les pressions des séquences qui leur correspondent.

Le dispositif selon l'invention peut comporter des caractéristiques additionnelles prises séparément et en combinaison, et notamment :
- l'un au moins des premier et second réservoirs pourra comporter un orifice adapté à l'introduction du fluide de culture dans un conduit de communication entre poches ;
- l'une au moins des parties supérieures des réservoirs pourra comporter une ouverture supérieure permettant le raccordement de la poche supérieure qu'elle loge à un dispositif d'alimentation et d'extraction de fluide de culture ;
- des premiers moyens de contrôle d'accès, pilotés par le module de commande pourront contrôler l'accès à la poche supérieure via l'ouverture supérieure ;
- chaque partie inférieure de réservoir pourra comporter une ouverture inférieure permettant le raccordement des moyens de liaison à la poche inférieure qu'elle loge, et des seconds moyens de contrôle d'accès, pilotés par le module de commande, pourront contrôler l'accès aux poches inférieures via les ouvertures inférieures ;
- les parties supérieure et inférieure des premier et second réservoirs pourront chacune comporter une entrée étanche, et les moyens de pressurisation pourront comprendre un premier circuit de pressurisation alimentant en fluide haute pression, sous le contrôle de vannes supérieures et inférieures pilotées par le module de commande, chaque partie supérieure et inférieure des premier et second réservoirs, via les entrées étanches ;
- les parties supérieure et inférieure des premier et second réservoirs pourront chacune comporter une entrée étanche, et les moyens de pressurisation pourront comprendre, d'une part, un premier circuit de pressurisation comportant une pompe à fluide (ou compresseur) et alimentant en fluide haute pression, sous le contrôle de vannes supérieure et inférieure pilotées par le module de commande, chaque partie supérieure et inférieure du premier réservoir, via les entrées étanches, et d'autre part, un second circuit de pressurisation comportant une pompe à fluide et alimentant en fluide haute pression, sous le contrôle de vannes supérieure et inférieure pilotées par le module de commande, chaque partie supérieure et inférieure du second réservoir, via les entrées étanches ;
- chaque circuit de pressurisation pourra également comprendre deux autres vannes coopérant respectivement avec les premier et seconds moyens de contrôle d'accès, lesquels sont alors avantageusement munis d'un conduit comportant une zone amincie flexible sur laquelle peut agir le fluide haute pression
- les moyens de pressurisation pourront également comprendre un troisième circuit de pressurisation alimentant en fluide basse pression, sous le contrôle des vannes supérieures et inférieures, chaque partie supérieure et inférieure des premier et second réservoirs, via les entrées étanches. En variante, les moyens de pressurisation pourront comprendre un troisième circuit de pressurisation alimentant en fluide basse pression, sous le contrôle desdites vannes supérieure et inférieure, chaque partie supérieure et inférieure du premier réservoir, via les entrées étanches, ainsi qu'un quatrième circuit de pressurisation alimentant en fluide basse pression, sous le contrôle des vannes supérieure et inférieure, chaque partie supérieure et inférieure du second réservoir, via les entrées étanches. Dans ce cas, chaque troisième et quatrième circuit de pressurisation est avantageusement raccordé aux autres vannes pour coopérer avec les premier et seconds moyens de contrôle d'accès ;
- au moins deux puits pourront être placés en série en communiquant par les moyens de liaison, l'un de ces puits étant raccordé au premier réservoir et l'autre puits étant raccordé au second réservoir. Un troisième puits, voire un quatrième ou un cinquième, pourra être placé en série entre les deux autres puits ;
- les puits pourront être réalisés sous la forme d'unités indépendantes assemblées les unes aux autres, ou bien ils pourront être agencés sur une plaque de culture ;
- chaque chambre de culture pourra comporter au moins une paroi transparente permettant l'observation des cellules ou tissus en cours de culture, par exemple sous un microscope ;
- chaque puits pourra être agencé de manière à définir deux chambres de culture superposées et séparées par un filtre.

L'invention concerne en outre une installation de culture de cellules et tissus comprenant au moins deux dispositifs du type décrit ci-avant, placés en parallèle, et comportant une unique unité de commande pilotant conjointement les unités de commande de ces dispositifs.

L'invention concerne également un procédé de culture de cellules et tissus. Ce procédé part d'une première étape connue dans laquelle on prévoit un dispositif de culture comportant au moins un puits de culture pour recevoir des cellules ou des tissus à cultiver, de premier et second réservoirs couplés au puits, dont l'un au moins est agencé pour recevoir un fluide de culture. Ledit procédé se caractérise par le fait que dans la première étape on loge dans chaque réservoir au moins une poche souple, dont l'une au moins peut recevoir le fluide de culture, et par le fait qu'il comprend une seconde étape dans laquelle on applique aux poches des pressions définies dans des première et seconde séquences de pressions choisies, pour gérer la circulation du fluide de culture dans le(s) puits.

Préférentiellement, dans la première étape on prévoit dans chaque réservoir une partie supérieure et une partie inférieure reliées entre elles par une partie intermédiaire étroite et logeant chacune une poche souple. Les poches supérieure et inférieure de chaque réservoir communiquent entre elles via la partie intermédiaire et les poches inférieures sont couplées au puits. Par ailleurs, dans la seconde étape on applique sur les poches supérieure et inférieure du premier réservoir des pressions définies par des premières séquences de pressions supérieures et inférieures choisies, respectivement, et sur les poches inférieure et supérieure du second réservoir des pressions définies par des secondes séquences de pressions inférieures et supérieures choisies, respectivement.

Deux principaux modes de mise en oeuvre de la seconde étape peuvent être envisagés selon le débit de fluide de culture requis. Dans un premier mode, dit « laminaire », plus particulièrement adapté aux faibles débits, on contraint le fluide de culture à monter dans la poche supérieure de l'un des réservoirs, de sorte qu'il se trouve placé à un niveau supérieur à celui de la poche inférieure de l'autre réservoir, puis on le laisse gagner cette poche inférieure de l'autre réservoir, en le forçant, par conséquent à traverser le(s) puits. On contraint alors le fluide à monter dans la poche supérieure, puis on effectue le transfert vers l'autre poche supérieure, dans le sens inverse. Ce va et vient est répété un nombre de fois choisi. Ce mode convient tout particulièrement aux cellules non adhérentes dans la mesure où, le flux de fluide de culture étant très faible et les cellules présentant malgré tout un coefficient non nul d'adhésion aux parois de la chambre de culture, ces cellules ne sont pas emportées par le flux de fluide d'une chambre de culture à l'autre.

Dans un second mode, dit « turbulent », plus particulièrement adapté aux forts débits, on applique en permanence une haute pression sur les deux poches supérieures, ou inférieures, et l'on applique sur les poches inférieures, ou supérieures, des séquences de pression en « opposition de phase », constituées d'alternances de périodes de haute et basse pressions. Dans une variante, on pourrait également envisager d'appliquer une même première séquence de pression simultanément sur les deux poches supérieure et inférieure d'un réservoir et une même seconde séquence de pression simultanément sur les deux poches supérieure et inférieure de l'autre réservoir, les première et seconde séquences étant en opposition de phase.

Bien entendu, on peut envisager de combiner ces différents modes entre eux.

D'autres caractéristiques et avantages de l'invention apparaîtront à l'examen de la description détaillée ci-après, et des dessins annexés, sur lesquels :
- la figure 1 est illustre de façon schématique, dans une vue en coupe transversale, un dispositif de culture selon l'invention, à plusieurs chambres,
- la figure 2 illustre de façon détaillée, dans une vue en coupe transversale, l'un des réservoirs de la figure 1,
- la figure 3 illustre de façon détaillée, dans une vue en coupe transversale, l'assemblage des deux poches souples du réservoir de la figure 2,
- les figures 4A et 4B illustrent schématiquement, dans des vues en coupe transversale, deux états des moyens de contrôle d'accès aux poches souples, dans un mode de réalisation préférentiel,
- la figure 5 illustre de façon schématique un bloc de gestion de la pressurisation d'un réservoir,
- la figure 6 est une vue schématique en perspective d'une installation de culture constituée de quatre dispositifs de culture placés en parallèle,
- les figures 7A et 7B sont des vues schématique, respectivement en perspective et du dessus, d'une plaque à puits pour une installation du type de celle illustrée sur la figure 6,
- les figures 8A à 8H sont des vues schématiques des principaux constituants d'un puits, avant et après assemblage,
- la figure 9 illustre schématiquement, dans une vue en coupe transversale, une variante de réalisation d'une structure multipuits,
- les figures 10A à 10H sont des vues schématiques illustrant les huit phases successives d'un aller-retour du fluide de culture dans un mode de circulation de type laminaire,
- la figure 11 illustre l'enchaînement des huit phases des figures 11,
- les figures 12A à 12D sont des vues schématiques illustrant les quatre phases successives d'un aller-retour du fluide de culture dans un mode de circulation de type turbulent,
- la figure 13 illustre l'enchaînement des quatre phases des figures 12,
- la figure 14 est une variante du mode turbulent illustré sur les figures 12 et 13.

Les dessins annexés sont, pour l'essentiel, de caractère certain. En conséquence, ils pourront non seulement servir à compléter l'invention, mais aussi contribuer à sa définition, le cas échéant.

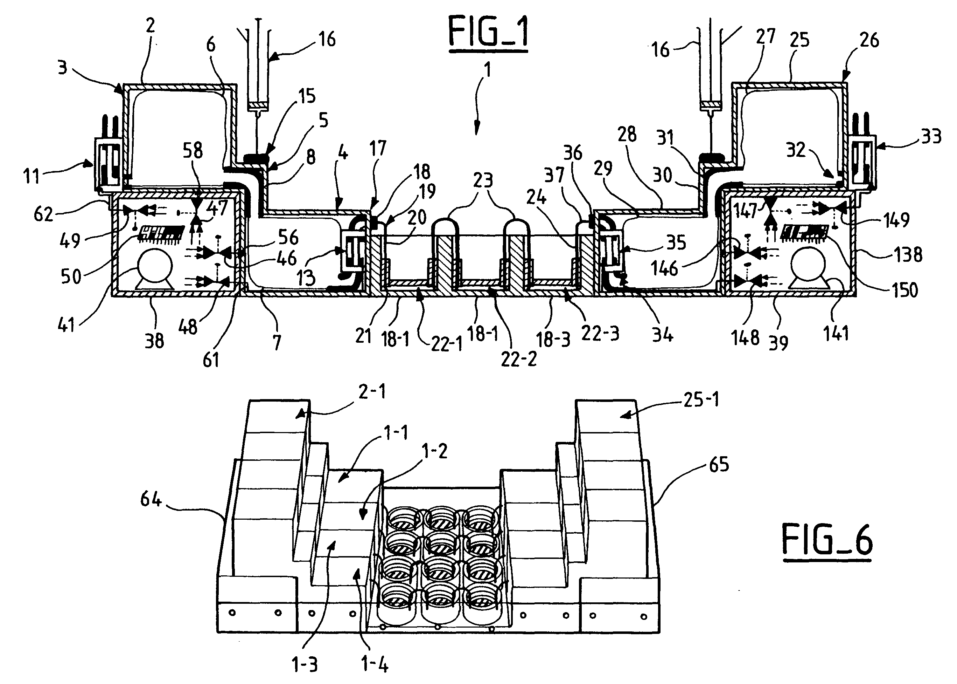

On se réfère tout d'abord à la figure 1 pour décrire un dispositif de culture de cellules et tissus, dans un mode de réalisation, non limitatif.

Le dispositif 1 illustré sur la figure 1 comprend tout d'abord un premier réservoir 2 comportant une partie supérieure 3 couplée à une partie inférieure 4 par une partie intermédiaire 5. Préférentiellement, ce réservoir est délimité par des parois rigides qui lui confèrent un volume constant.

Dans l'exemple illustré, la partie supérieure 3 du réservoir loge une poche souple supérieure 6. De même, la partie inférieure 4 loge une poche souple inférieure 7 qui est raccordée à la poche supérieure 6 par un conduit 8 logé dans la partie intermédiaire 5, étroitement, de sorte que les parties supérieure 3 et inférieure 4 du premier réservoir 2 soient isolées l'une de l'autre.

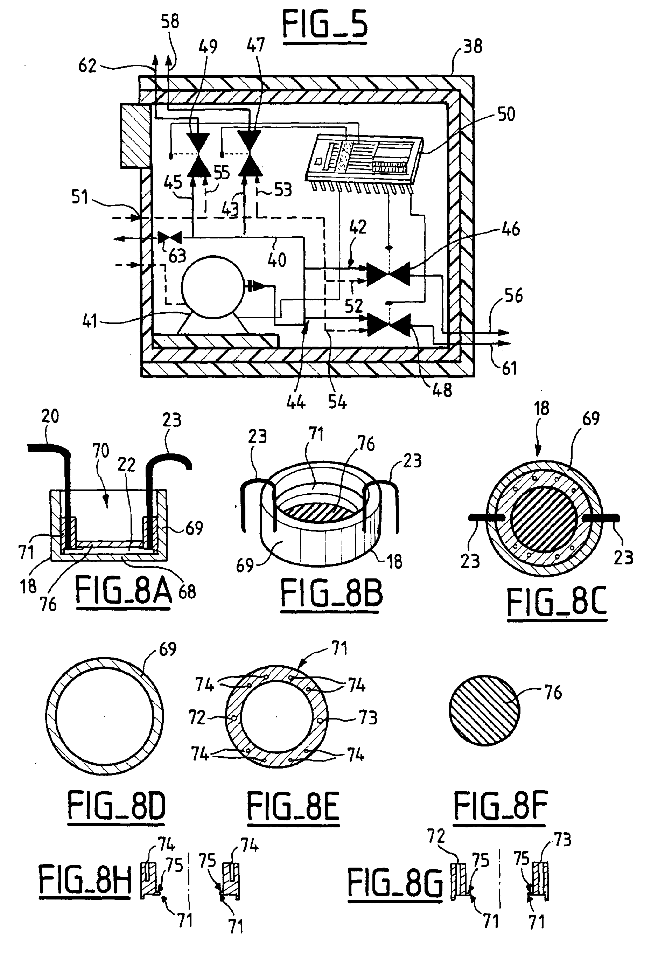

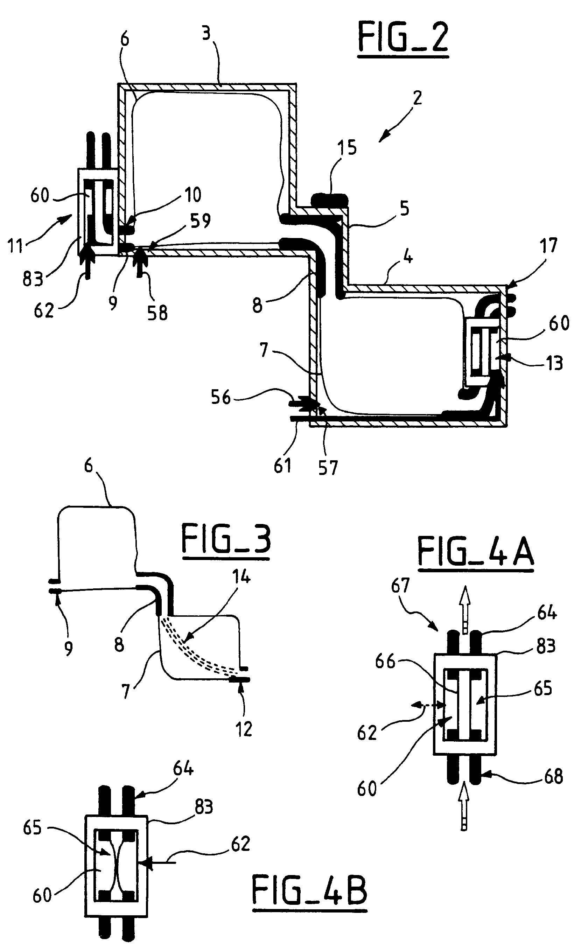

Comme cela est mieux illustré sur les figures 2 et 3, la poche supérieure 6 comporte une entrée/sortie 9 adaptée de manière à pouvoir coopérer, de façon étanche, avec une ouverture supérieure 10 formée dans l'une des cloisons de la partie supérieure 3 du premier réservoir 2. Ainsi, la poche supérieure 6 peut être raccordée à des moyens de contrôle d'accès supérieurs 11, eux-mêmes raccordés à un module d'alimentation en gaz ou en fluide de culture (non représenté).

De même, la poche inférieure 7 comporte une entrée/sortie 12 adaptée de manière à coopérer avec une ouverture formée dans la paroi de la partie inférieure 4 du premier réservoir 2, ou bien, comme cela est illustré sur la figure 2, de manière à coopérer avec des moyens de contrôle d'accès 13 logés à l'intérieur de la partie inférieure 4 du réservoir 2.

Préférentiellement, comme cela est illustré sur la figure 3, la poche inférieure 7 peut comporter deux membranes sensiblement rigides 14 évitant son écrasement complet lorsqu'elle est soumise à des pressions très élevées, ce qui nuirait à la bonne circulation du fluide de culture.

Egalement de préférence, le premier réservoir 2 comporte, dans la partie intermédiaire 5, une autre ouverture 15 permettant l'injection, ou l'extraction, manuelle ou automatique, d'un liquide ou d'un gaz à l'intérieur du conduit 8. Il s'agira, de préférence, d'une ouverture 15 équipée d'un septum, particulièrement approprié lorsque le dispositif d'injection ou d'extraction est une seringue 16 munie d'une aiguille.

Egalement de préférence, les poches supérieure 6 et inférieure 7 sont réalisées dans un matériau poreux, au moins dans le sens allant de l'extérieur vers l'intérieur. Il pourra s'agir de poches en silicone, ou en polydimethylsiloxane (PDSM), ou encore en polytetrafluorethylène (PTFE), ou encore en polymères dimethyl et methylvinyl siloxanes. Cela permet, en effet, des échanges gazeux entre le fluide de culture qui se trouve logé à l'intérieur des poches souples et le gaz qui se trouve piégé à l'intérieur des parties supérieure 3 et inférieure 4 du premier réservoir 2.

Dans l'exemple illustré sur la figure 1, la poche inférieure 7 communique avec des puits de culture 18-1 à 18-3, via les moyens de contrôle d'accès 13 et l'ouverture inférieure 17 formée dans la paroi du réservoir.

Plus précisément, les moyens de contrôle d'accès 13 comportent une extrémité creuse 18 dans laquelle est introduite une extrémité 19 d'un moyen de liaison 20, réalisé sous la forme d'un conduit (ou tube), et dont l'extrémité opposée 21 débouche dans la chambre de culture 22-1 du premier puits 18-1. Cette première chambre de culture 22-1 communique avec la seconde chambre de culture 22-1 logée dans le second puits 18-2 par l'intermédiaire d'un autre moyen de liaison 23, également réalisé sous la forme d'un conduit (ou tube). De même, la seconde chambre de culture 22-2 communique avec la troisième chambre de culture 22-3 logée dans le troisième puits 18-3 par l'intermédiaire d'un autre moyen de liaison 23 réalisé sous la forme d'un conduit (ou tube). Enfin, dans cet exemple, un dernier moyen de liaison 24 assure la communication entre la troisième chambre de culture 22-3 et un second réservoir 25, qui va être décrit maintenant.

Ce second réservoir 25 est, de préférence, sensiblement identique au premier réservoir 2 qui a été décrit précédemment en référence aux figures 1 à 4. Il comporte, par conséquent, dans cet exemple, une partie supérieure 26 dans laquelle se trouve logée une poche souple supérieure 27, une partie inférieure 28 dans laquelle se trouve logée une poche souple inférieure 29 et une partie intermédiaire 30 étroite qui loge un conduit 31 couplant la poche supérieure 27 à la poche inférieure 29. Ce conduit 31 est également logé, de façon étroite, dans la partie intermédiaire 30, de sorte que la partie supérieure 26 soit isolée, sur le plan de l'étanchéité au gaz, de la partie inférieure 28.

La poche supérieure 27 comporte une entrée/sortie 32 adaptée, raccordée à des moyens de contrôle d'accès 33, lesquels, tout comme les moyens de contrôle d'accès 11, peuvent être raccordés à un dispositif d'alimentation en fluide ou gaz, ou à un extracteur (non représenté). De même, la poche inférieure 29 comporte une entrée/sortie 34 qui, dans l'exemple illustré, est raccordée à des moyens de contrôle d'accès 35, logés à l'intérieur de la partie inférieure 28 du second réservoir 25.

De préférence, le second réservoir 25 comporte également, dans sa partie intermédiaire 30, une autre ouverture permettant l'injection, ou l'extraction, manuelle ou automatique, d'un liquide ou d'un gaz à l'intérieur du conduit 31. II s'agira, de préférence, d'une ouverture équipée d'un septum.

Dans cet exemple, les moyens de contrôle d'accès 35 comportent une extrémité creuse 36 à laquelle est raccordée l'extrémité 37 du conduit 24.

Un circuit est ainsi créé entre la poche supérieure 6 du premier réservoir 2 et la poche supérieure 27 du second réservoir 25, via les chambres de culture 22-i (i = 1 à 3, dans cet exemple) et via les moyens de liaison 20, 23 et 24.

Pour gérer les volumes internes des poches supérieures 6 et 27 et des poches inférieures 7 et 29, le dispositif selon l'invention comporte des moyens de pressurisation qui vont être décrits maintenant en référence aux figures 1 et 5.

Dans le mode de réalisation préférentiel illustré, chaque réservoir comporte ses propres moyens de pressurisation logés dans un boîtier 38 ou 39. Ces boîtiers étant sensiblement identiques, seul celui couplé au premier réservoir 2 sera décrit ci-après.

Le boîtier 38 comporte un premier circuit de pressurisation 40, haute pression, comportant une pompe 41, de préférence à débit variable.

Ce premier circuit haute pression 40 comporte une première voie 42 destinée à alimenter en air pressurisé l'intérieur de la partie inférieure 4 du premier réservoir 2, de manière à gérer le volume de la poche inférieure 7.

Le premier circuit haute pression comporte une seconde voie 43 destinée à alimenter l'intérieur de la partie supérieure 3, de manière à gérer le volume de la poche supérieure 6.

Dans le mode de réalisation illustré, le premier circuit haute pression 40 comporte également de troisième 44 et quatrième 45 voies destinées respectivement à gérer l'état des moyens de contrôle d'accès 11 et 13, sur lesquels on reviendra plus loin.

Chacune de ces première 42, seconde 43, troisième 44 et quatrième 45 voies comporte une électrovanne, respectivement référencée 46 à 49.

Toutes ces électrovannes 46 à 49 et la pompe à fluide de pressurisation 41 sont contrôlées par une unité de contrôle 50, réalisée sous la forme d'un microprocesseur (ou microcontrôleur). Le bloc de pressurisation 38 comporte également un second circuit de pressurisation 51 (matérialisé par des traits en pointillés) qui débouche à l'extérieur, de manière à offrir une basse pression, équivalente à la pression atmosphérique. Ce second circuit de pressurisation 51 comporte quatre voies 52 à 55 respectivement raccordées aux électrovannes 46 à 49, lesquelles sont par conséquent de type trois voies (deux d'entrée et une de sortie).

De la sorte, le microcontrôleur 50 est capable d'appliquer des basses pressions ou des hautes pressions, selon les besoins, dans les parties supérieures 3 et inférieures 4 du premier réservoir 2.

La sortie de l'électrovanne 48 de la première voie 44 alimente, via un conduit 56, une ouverture 57 formée dans la paroi de la partie inférieure 4 du premier réservoir 2. De même, la sortie de l'électrovanne 47 alimente, via un conduit 58 une ouverture 59 formée dans la paroi de la partie supérieure 3 dudit premier réservoir 2. Enfin, les sorties des électrovannes 48 et 49 alimentent respectivement des logements 60, que comprennent les moyens de contrôle d'accès 11 et 13, via des conduits 61 et 62, respectivement.

Ces logements sont détaillés sur les figures 4A et 4B. En fait, dans le mode de réalisation illustré, les moyens de contrôle d'accès 11, 13, 33 et 35, comprennent un boîtier 63 fermé, et évidé, de manière à délimiter le logement interne 60 et à recevoir un tube 64 comportant, dans une partie centrale 65, logée à l'intérieur du logement 60, une partie amincie 66, d'une grande souplesse. Les deux extrémités 67 et 68 de ce tube 64 débouchent à l'extérieur du logement 60 par des ouvertures formées dans le boîtier 83, de sorte qu'elles puissent être raccordées soit à une entrée/sortie 9 ou 12 d'une poche souple 6 ou 7, soit à l'extrémité 19 ou 37 d'un moyen de liaison 20 ou 24, soit encore à un dispositif d'alimentation ou d'extraction de gaz ou de liquide (non représenté). Mais il ne s'agit que d'un exemple de réalisation parmi d'autres, tels que des commutateurs ou des vannes.

En appliquant une haute pression sur la partie amincie 66 du tube 64, comme illustré sur la figure 4B, on provoque un écrasement des parois amincies qui interdit le passage, de tout fluide. En d'autres termes, selon que la pression appliquée dans le logement 60 sur la zone amincie 66 du tube 64 est haute ou basse, le moyen de contrôle d'accès 11 ou 13 se trouve dans un état non passant ou passant (ou encore fermé ou ouvert), si bien qu'il agit comme un commutateur. Cette commutation n'est pas obligatoirement de type tout ou rien, bien que cela soit préférable. En effet, selon l'intensité de la pression appliquée sur la zone amincie 66, on peut obtenir un écrasement partiel ou total.

De préférence, le premier circuit 40 comporte, également, une électrovanne additionnelle 63 permettant de régler le débit du fluide de pressurisation. Cette électrovanne est également pilotée par le microcontrôleur 50.

D'autre part, le bloc de pressurisation comporte, de préférence, une interface de communication, par exemple de type RS232, permettant le raccordement dudit boîtier à un micro-ordinateur destiné à programmer le microcontrôleur 50.

Comme indiqué précédemment, dans le mode de réalisation illustré sur la figure 1, le dispositif selon l'invention comporte un autre bloc de pressurisation 138 sensiblement identique au bloc 38 qui vient d'être décrit en référence à la figure 5. Les références des composants de ce bloc 13 présentent les mêmes références que celles des éléments du bloc 38, augmentées de la valeur 100. Ainsi, la pompe à fluide de pressurisation porte la référence 141, les différentes électrovannes du circuit de pressurisation portent les références 146 à 149 et le microcontrôleur porte la référence 150.

Préférentiellement, les deux blocs de pressurisation sont indépendants l'un de l'autre.

Ce mode de réalisation est actuellement préféré, dans la mesure où chaque bloc de pressurisation 38, 138 peut être intégré sous la partie supérieure 3, 26 d'un premier 2 ou second réservoir 25, en réalisant un assemblage modulaire compact. De plus, cela simplifie notablement les circuits de pressurisation, évitant notamment les problèmes de raccordement.

Bien entendu, dans une variante de réalisation, le dispositif selon l'invention pourrait ne comprendre qu'un unique bloc de pressurisation gérant la pression appliquée sur chacune des poches souples des réservoirs. Dans une telle variante, on pourrait n'utiliser qu'un unique microcontrôleur pour gérer l'intégralité des électrovannes du circuit de pressurisation. Dans une autre variante, on pourrait conserver un bloc de pressurisation pour chaque réservoir, mais n'utiliser qu'une unique pompe à fluide et/ou un unique micro-contrôleur.

Comme cela est illustré, schématiquement, sur la figure 6, il est possible de disposer en parallèle une multiplicité de dispositifs 1, de manière à constituer une installation de culture de cellules et tissus, à grand rendement. Dans cet exemple, l'installation comporte quatre dispositifs parallèles 1-1 à 1-4, chaque dispositif 1-i (ici i = 1 à 4) comportant chacun trois puits de culture 18-j (ici j = 1 à 3) en série. Ces dispositifs sont ici indépendants, mais ils pourraient coopérer ensemble, par exemple du fait de moyens de liaison couplant certains de leurs puits.

De préférence, dans ce type d'installation, les moyens de pressurisation de chaque dispositif 1-i sont logés dans deux boîtiers 64 et 65 destinés respectivement au premier réservoir 2-i et 25-i, préférentiellement, chaque boîtier 64, 65 loge autant de blocs de pressurisation 38, 138 qu'il y a de dispositifs, chaque bloc étant avantageusement indépendant du bloc gérant le réservoir du dispositif voisin. Mais, dans une variante, on pourrait envisager que le boîtier 64 comporte un unique microcontrôleur permettant de piloter l'intégralité des électrovannes gérant les différents accès aux parties supérieures et inférieures de chaque réservoir. Il pourrait également n'y avoir qu'une unique pompe.

Dans une telle installation, le nombre de dispositifs assemblés en parallèle pourra varier selon les besoins.

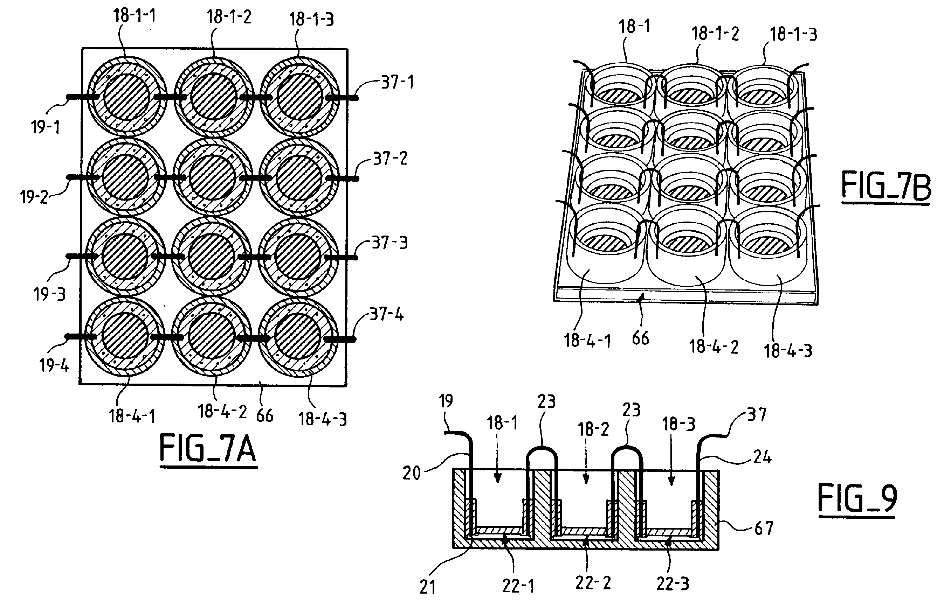

Dans une installation selon l'invention, tout comme dans un dispositif selon l'invention, les puits de culture 18-j peuvent être montés en série sur une plaque support, comme illustré sur les figures 7A et 7B, ou bien directement formés par évidement d'un bloc massif épais 67 (comme illustré sur les figures 1 et 9).

Dans le premier exemple (figures 7), la plaque pourra comporter des logements pour recevoir chaque puits 18-i-j (ici i = 1 à 4, et j = 1 à 3).

Dans le second exemple de réalisation, les puits de culture des dispositifs pourront être réalisés dans des blocs massifs indépendants, ou bien dans un bloc unique.

On se réfère maintenant aux figures 8A à 8H pour décrire un mode de réalisation préférentiel des puits de culture.

Comme illustré sur la figure 8A, un puits, de type indépendant, est délimité par des parois, dont une paroi de fond 68, de préférence plane et une paroi latérale 69, de préférence cylindrique, bien que tout autre type de forme pourrait être envisagé, selon les besoins. Ces parois 68 et 69 délimitent une cavité 70 au fond de laquelle se trouve placé un anneau souple 71 (voir figures 8E et 8G) dans lequel se trouvent formés deux orifices traversant 72 et 73, destinés à permettre le passage des moyens de liaison 20, 23 ou 24. Ces moyens de liaison sont de préférence rigides, et présentent un diamètre externe légèrement supérieur au diamètre interne des orifices, de manière à assurer une étanchéité de qualité.

De préférence, cet anneau souple 71 présente les propriétés d'un septa, de manière à permettre des injections multiples, à l'aide d'aiguilles, à l'intérieur de la chambre de culture 22, et sans que cela ne nuise à l'étanchéité. Il pourra s'agir, par exemple d'un anneau en silicone comportant des orifices préformés 74 délimitant des points d'injection (voir figure 8H).

L'anneau souple 71 comporte dans sa partie inférieure, côté interne, un bourrelet 75, sensiblement circulaire, destiné à supporter un couvercle 76, de préférence transparent (voir figure 8F).

Ce couvercle 76 est destiné à être immobilisé dans la cavité 70, à une distance choisie du fond du puits, délimité par la paroi 68, de manière à constituer la chambre de culture 22 dans laquelle doit s'effectuer la culture des cellules ou tissus, de façon étanche.

Le fond du puits, délimité par la face supérieure de la paroi 68, peut être traité préalablement en fonction des besoins spécifiques d'une lignée cellulaire. Un tel traitement peut être chimique ou physique. Par ailleurs, une lamelle, avec ou sans traitement spécifique, peut être déposée sur cette paroi 68, de manière à permettre un ancrage des cellules.

D'autres éléments bio-compatibles, ou non, peuvent être introduits dans la chambre 22 pour permettre l'adhésion des cellules ou tissus, ou l'étude des interférences entre cet élément et la culture déjà existante. Il pourra s'agir, par exemple, de gel, de collagène, de filtre, ou tout autre support.

Comme indiqué précédemment, il est particulièrement avantageux que le couvercle 76 soit transparent, de manière à permettre une observation microscopique, en direct, pendant toute la durée de la culture. A cet effet, les différents constituants du dispositif, que ce soit les réservoirs, les blocs de pressurisation, ou même les puits, sont miniaturisés pour permettre leur installation sous un objectif de microscope.

De préférence, le couvercle 76 peut être retiré, afin d'être changé, ou bien de manière à permettre l'enlèvement des cellules ou tissus cultivés, dans un environnement stérile.

En vue d'éviter des changements de pression à l'intérieur de la chambre de culture 22, lors de l'enlèvement du couvercle 76, une aiguille, ou tout autre dispositif, peut être introduite dans un ou plusieurs des orifices 74 de manière à établir une communication entre l'intérieur de la chambre de culture 22 et le milieu extérieur environnant.

Après enlèvement du couvercle 76, la culture peut être traitée manuellement ou automatiquement, selon les différents procédés connus de l'homme de l'art.

Par ailleurs, les orifices 74 peuvent être utilisés de manière à établir des communications multiples, directes, automatiques ou manuelles dans la chambre de culture 22, en vue de toute intervention fluidique autre que celle assurée par les moyens de liaison 20, 23 et 24 qui alimentent les chambres en fluide de culture. A titre d'exemple, l'inoculation initiale des cellules peut être directement réalisée par un ou plusieurs orifices 74.

Ainsi, des traitements différents peuvent être appliqués dans des chambres de culture d'une même série. Ces orifices 74 peuvent être également utilisés pour connecter une chambre à plusieurs autres chambres d'une même série ou d'une autre série (lorsque plusieurs dispositifs sont installés en parallèle dans une installation).

De préférence, les tubes (ou conduits), formant les moyens de liaison, sont réalisés dans un matériau élastomère souple pour permettre de bloquer la circulation de fluide de culture par écrasement, par exemple à l'aide d'une pince.

Dans une variante, certains des puits pourront être agencé de manière à définir deux chambres de culture superposées et séparées par un filtre.

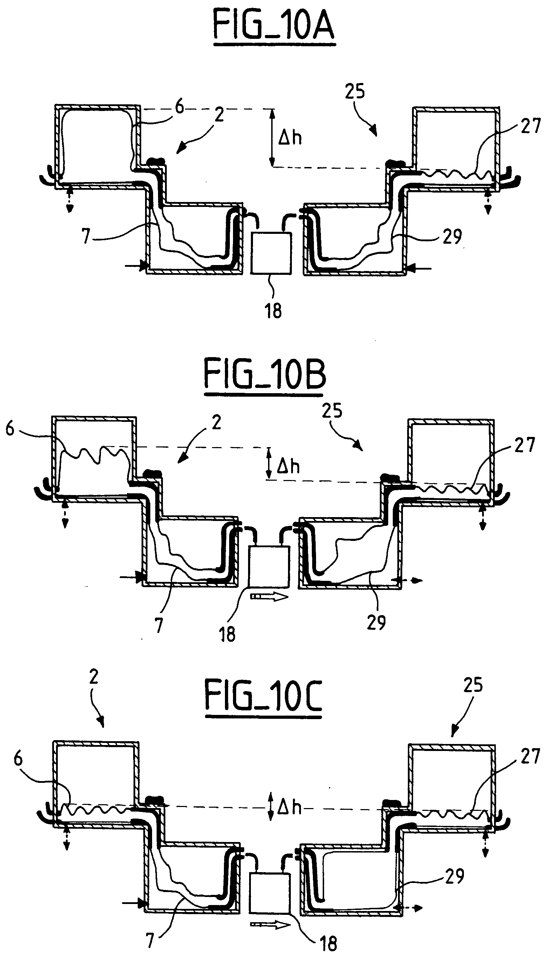

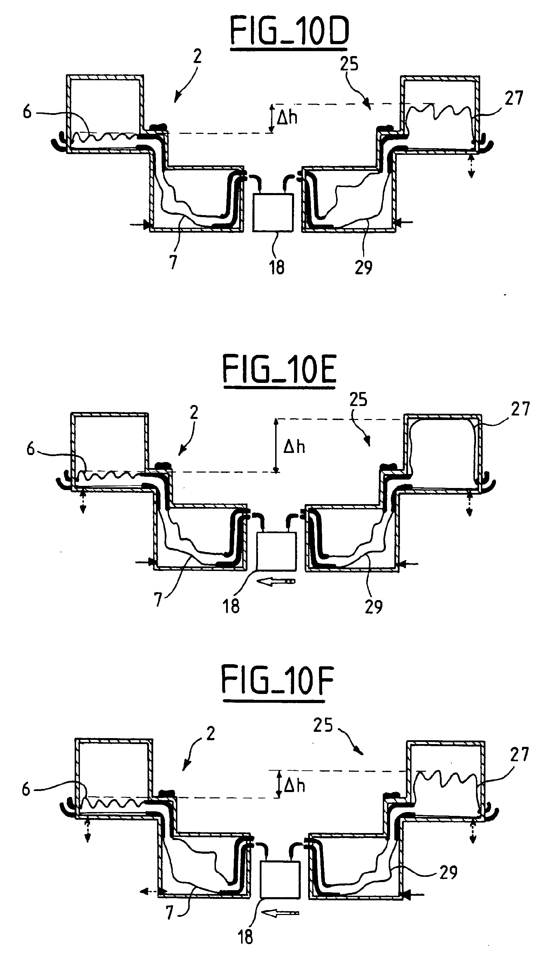

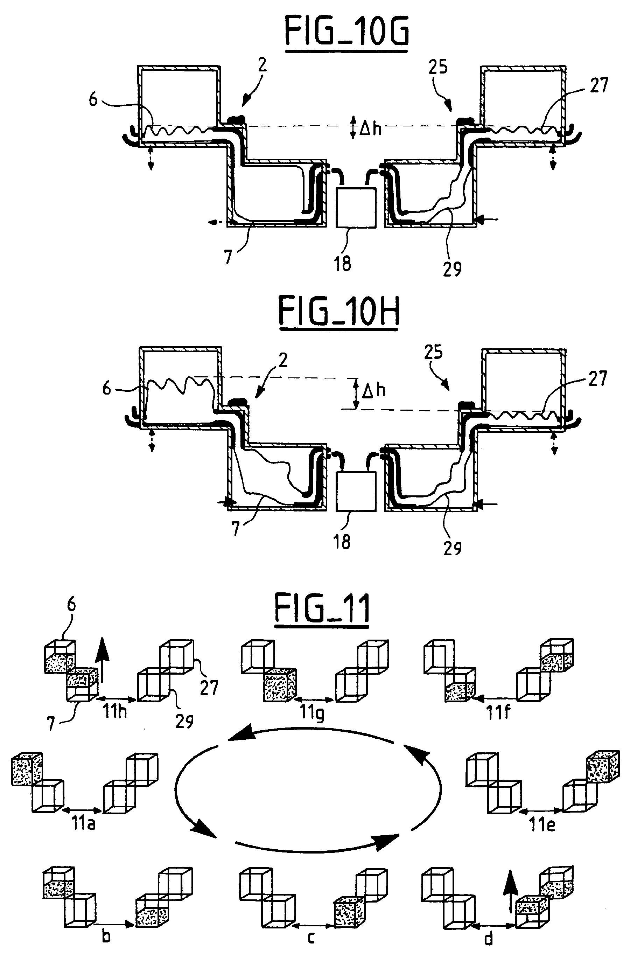

On se réfère maintenant aux figures 10 et 11 pour décrire un premier mode de fonctionnement du dispositif et de l'installation selon l'invention. Ce premier mode peut être appelé mode laminaire. Il consiste à faire remonter le fluide de culture dans la poche supérieure de l'un des deux réservoirs de manière à instaurer une différence de hauteur entre cette poche supérieure et les poches inférieures des deux réservoirs, puis à laisser s'écouler, par gravitation, le fluide de culture du réservoir supérieur vers les réservoirs inférieurs, et à faire remonter le fluide de culture vers la poche supérieure de l'autre réservoir. On recommence, dans l'autre sens ("sens retour"), les mêmes opérations pour faire un cycle (ou "aller-retour") entre les deux réservoirs, via le puits. Le nombre de cycles est choisi en fonction du type de culture effectuée à l'intérieur des puits 18.

Les microcontrôleurs 50, 150 des moyens de pressurisation sont programmés, ou configurés, pour appliquer sur chaque poche supérieure et inférieure qu'ils gèrent des pressions définies par des séquences de pression.

Ces séquences de pression sont, de préférence, préalablement mémorisées dans une mémoire réinscriptible du microcontrôleur. Dans ce cas, la programmation (ou en d'autres termes la mémorisation des différentes séquences) est avantageusement effectuée via l'interface RS232 que l'on raccorde à un micro-ordinateur, ou analogue. Mais, le microcontrôleur peut être équipé d'une mémoire non réinscriptible, et dans ce cas il ne peut pas être reprogrammé avec de nouvelles séquences.

Le mode de circulation laminaire peut être décomposé en cycles de quatre étapes illustrées sur les figures 10A à 10H. Chaque étape correspond, pour chaque poche de chaque réservoir, à une période de la séquence associée.

Dans la première étape illustrée sur les figures 10H et 10A, le fluide de culture qui vient d'être injecté dans le premier réservoir 2 est contraint à gagner la poche supérieure 6. Pour ce faire, on applique une basse pression sur la poche supérieure 6 du premier réservoir 2, des hautes pressions sur les poches inférieures 7 et 29, tandis que l'on applique une basse pression sur la poche supérieure 27 du second réservoir 25. Le fluide de culture est donc contraint à quitter la poche supérieure 27 du second réservoir 25 pour gagner la poche supérieure du premier réservoir 2, via les poches inférieures 29 et 7. En d'autres termes, la poche supérieure 27 du second réservoir 25 se dégonfle tandis que la poche supérieure 6 du premier réservoir 2 se gonfle en se remplissant de fluide de culture.

Dans la seconde étape illustrée sur les figures 10B et 10C, le fluide de culture qui vient d'être regroupé dans la poche supérieure 6 du premier réservoir 2 s'écoule par gravitation de la poche supérieure 6 vers la poche inférieure 7 du premier réservoir puis la poche inférieure 29 du second réservoir 25. Pour ce faire, on applique une basse pression sur la poche supérieure 6 du premier réservoir 2 et une haute pression sur la poche inférieure 7 de ce même réservoir, tandis que l'on applique des basses pressions sur les poches inférieure 29 et supérieure 27 du second réservoir 25. La poche supérieure 6 se dégonfle donc, tandis que la poche inférieure 29 du second réservoir 25 se gonfle en se remplissant de fluide de culture.

Dans la troisième étape illustrée sur les figures 10D et 10E, le fluide de culture qui vient d'être regroupé dans la poche inférieure 29 du second réservoir 25 est contraint à gagner la poche supérieure 27. Pour ce faire, on applique une basse pression sur la poche supérieure 6 du premier réservoir 2, des hautes pressions sur les poches inférieures 7 et 29, et une basse pression sur la poche supérieure 27 du second réservoir 25. La poche inférieure 29 se dégonfle donc, tandis que la poche supérieure 27 du second réservoir 25 se gonfle en se remplissant de fluide de culture.

Dans la quatrième étape illustrée sur les figures 10F et 10G, le fluide de culture qui vient d'être regroupé dans la poche supérieure 27 du second réservoir 25 s'écoule par gravitation de la poche supérieure 27 vers la poche inférieure 7 du premier réservoir 2. Pour ce faire, on applique des basses pressions sur les poches supérieures 6 et 27 et sur la poche inférieure 7 du premier réservoir, tandis que l'on applique une haute pression sur la poche inférieure 29 du second réservoir 25. La poche supérieure 27 se dégonfle donc, tandis que la poche inférieure 7 du premier réservoir 2 se gonfle en se remplissant de fluide de culture. Cela met fin au cycle aller-retour.

Sur la figure 10H se trouve illustrée le début de la première étape.

Les quatre étapes du mode laminaire sont regroupées sous la forme d'un cycle « aller-retour » sur la figure 11. Le nombre de cycles successifs est choisi en fonction du type de culture effectué.

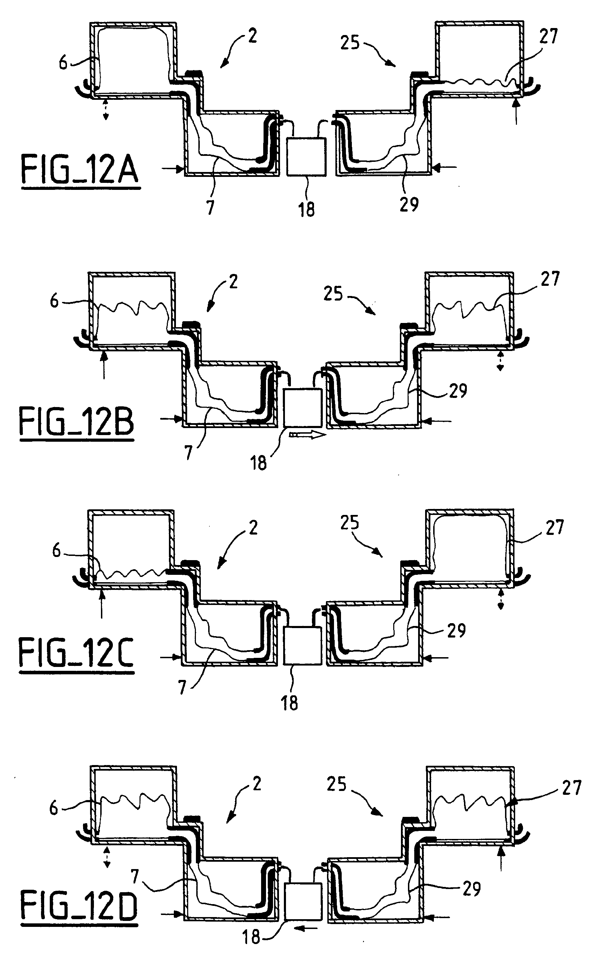

On se réfère maintenant aux figures 12A à 12D pour décrire un second mode de fonctionnement d'un dispositif ou d'une installation selon l'invention.

Dans ce mode de fonctionnement dit « turbulent », une haute pression est appliquée en permanence sur les poches inférieures 7 et 29 des premier 2 et second 25 réservoirs. En d'autres termes, les première et seconde séquences supérieures des poches supérieures des premier et second réservoirs sont constituées d'une succession de quatre périodes de basse pression. Ce mode ne comprend que deux étapes.

Dans la première étape illustrée sur les figures 12A et 12D, on applique une basse pression sur la poche supérieure 6 du premier réservoir 2 et une haute pression sur la poche supérieure 27 du second réservoir 25. Le fluide est donc contraint à gagner la poche supérieure 7 du premier réservoir 2, si bien que la poche supérieure 27 se dégonfle tandis que la poche supérieure 6 se gonfle.

Dans la seconde étape illustrée sur les figures 12B et 12C, on applique une haute pression sur la poche supérieure 6 du premier réservoir 2 et une basse pression sur la poche supérieure 27 du second réservoir 25. Le fluide est donc contraint à gagner la poche supérieure 27 du second réservoir 25, si bien que la poche supérieure 6 se dégonfle tandis que la poche supérieure 27 se gonfle.

Sur la figure 12D se trouve illustrée le début de la première étape.

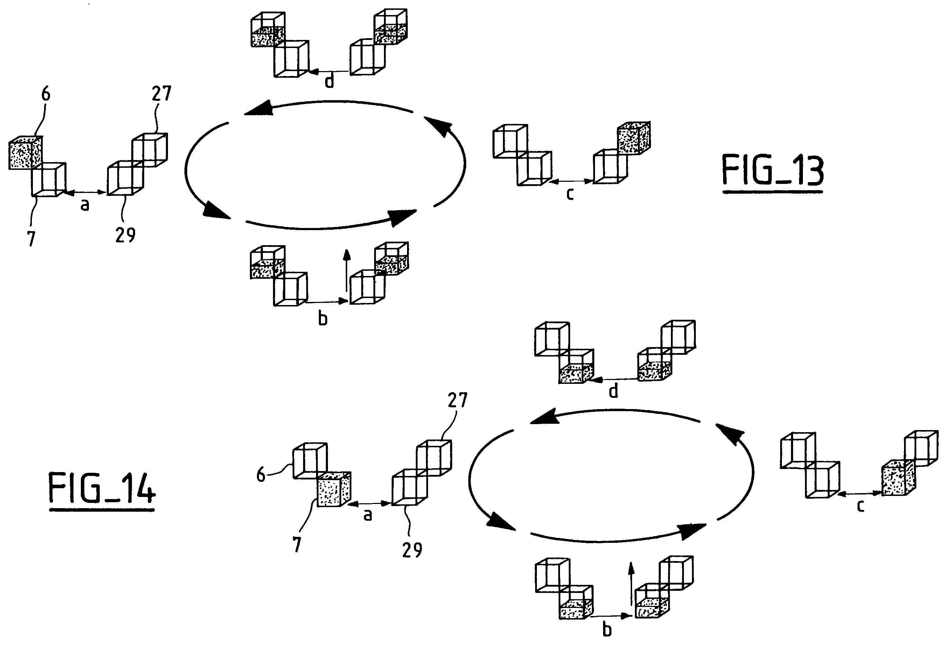

Les deux étapes du mode turbulent sont regroupées sous la forme d'un cycle « aller-retour » sur la figure 13. Le nombre de cycles successifs est choisi en fonction du type de culture effectué.

Sur la figure 14 se trouve représentée une variante de cycle « aller-retour» (entre les deux réservoirs) du second mode de fonctionnement turbulent. Dans cette variante, la haute pression n'est plus maintenue constamment sur les deux poches inférieures 7 et 29, mais sur les deux poches supérieures 6 et 27. Cela permet d'obtenir une circulation du fluide de culture très rapide entre les deux poches inférieures 7 et 29, étant donné que ledit fluide ne peut pas remonter, du fait des hautes pressions dans les poches supérieures 6 et 27.

Dans cette variante, on prévoit toujours deux étapes.

Dans la première étape (a et d), on applique une haute pression sur les deux poches supérieures 6 et 27 et sur la poche inférieure 29 du second réservoir, tandis que l'on applique une basse pression sur la poche inférieure 7 du premier réservoir 2.

Dans la seconde étape (b et c), on applique une haute pression sur les deux réservoirs supérieurs 6 et 27 ainsi que sur le réservoir inférieur 7 du premier réservoir 2, tandis que l'on applique une basse pression sur la poche inférieure 29 du second réservoir 25. A la fin de cette étape, on peut recommencer la première étape (c et a).

Le nombre de cycles successifs est également choisi en fonction du type de culture effectué.

Dans une autre variante du mode de fonctionnement turbulent (non illustrée), la première séquence appliquée à chaque poche du premier réservoir consiste en une alternance de premières périodes de haute pression et de secondes périodes de basse pression, et la seconde séquence appliquée à chaque poche du second réservoir consiste en une alternance de premières périodes de basse pression et de secondes périodes de haute pression.

Les deux modes de fonctionnement, laminaire et turbulent, qui viennent d'être décrits, ainsi que la variante, ne sont que quelques-uns des nombreux exemples qui peuvent être envisagés. Ainsi, il serait possible de combiner des cycles de fonctionnement turbulent avec des cycles de fonctionnement laminaire.

Ces différents modes de fonctionnement forment chacun une étape de procédé selon l'invention, dont une étape préalable consiste en l'utilisation de l'un des dispositifs décrits ci-avant. En d'autres termes, l'invention concerne également un procédé comprenant une première étape dans laquelle on prévoit l'un des dispositifs selon l'invention, et une seconde étape dans laquelle on applique à ce dispositif l'un des modes de fonctionnement laminaire ou turbulent, ou bien une combinaison de ceux-ci.

Dans cette seconde étape, on peut choisir des durées sensiblement identiques pour les premières périodes des premières et secondes séquences, et des durées sensiblement identiques pour les secondes périodes des premières et secondes séquences.

Par ailleurs, dans cette seconde étape, la première séquence supérieure appliquée à la poche supérieure du premier réservoir pourra consister en une suite répétée un nombre de fois choisi de première, seconde, troisième et quatrième périodes de basse pression, tandis que, d'une première part, la première séquence inférieure appliquée à la poche inférieure du premier réservoir consiste en une suite répétée ledit nombre de fois choisi d'une première période de haute pression, d'une seconde période de haute pression, d'une troisième période de haute pression et d'une quatrième période de basse pression, d'une deuxième part, la seconde séquence inférieure appliquée à la poche inférieure du second réservoir consiste en une suite répétée ledit nombre de fois choisi d'une première période de haute pression, d'une seconde période de basse pression, d'une troisième période de haute pression et d'une quatrième période de haute pression, et d'une troisième part, la seconde séquence supérieure appliquée à la poche supérieure du second réservoir consiste en une suite répétée ledit nombre de fois choisi d'une première, seconde, troisième et quatrième périodes de basse pression. Préférentiellement, dans ce cas, lors de la seconde étape les durées des premières périodes des premières et secondes séquences supérieure et inférieure sont sensiblement identiques, les durées des secondes périodes des premières et secondes séquences supérieure et inférieure sont sensiblement identiques, les durées des troisièmes périodes des premières et secondes séquences supérieure et inférieure sont sensiblement identiques, et les durées des quatrièmes périodes des premières et secondes séquences supérieure et inférieure sont sensiblement identiques.

L'invention s'applique à de très nombreux types de cellules et tissus, tels que notamment :
- les cellules intestines : Intestine 407, Caco-2, Colo 205, T84, SW 1116, WiDr, HT 29, HT 115, HT 55 ;
- les cellules endothéliales : HAOSMC (de l'acronyme anglais Human Aortic Smooth Muscle Cells) ;
- les cellules épidermales : NHEK-Neopooled (Human Epidermal Keratinocyte Neonatal), Equine Dermis ;
- les cellules cancéreuses : HeLa, CHO-K1 ;
- les cellules fibroplastiques de type intestinales : CCD-18Co
- les cellules fibroplastiques de type MRC-5, 3T3, Wi-38 ;
- les myélomes : SP2O-Ag14, P3X63 Ag8 653, MPC11;
- les hybridomes ;
- les cellules d'insectes : SF9.

Cette liste n'est en aucun cas exhaustive; il ne s'agit que d'exemples.

L'invention ne se limite pas aux modes de réalisation de dispositif, d'installation et de procédé décrits ci-avant, seulement à titre d'exemple, mais elle englobe toutes les variantes que pourra envisager l'homme de l'art dans le cadre des revendications ci-après.

## Revendications

1. Dispositif de culture de cellules et tissus du type comprenant au moins un puits de culture (18-i) agencé pour définir une chambre (22-i) propre à recevoir des cellules ou des tissus à cultiver, de premier (2) et second (25) réservoirs, l'un au moins de ces deux réservoirs étant agencé pour recevoir un fluide de culture, et des moyens de liaison (20,23,24) couplés audit puits et auxdits premier et second réservoirs pour permettre la circulation du fluide de culture d'un réservoir à l'autre via ledit puits,
caractérisé en ce que chaque réservoir (20,25) loge au moins une poche souple (6,7 ;27,29), l'une au moins des poches de ces réservoirs étant propre à recevoir le fluide de culture, et en ce que ledit dispositif comprend au moins un module de commande (50,150) propre à fournir de première et/ou de seconde séquences de pressions externes à appliquer, respectivement, sur les poches des premier et second réservoirs, et des moyens de pressurisation (38,40-49,51-56,61-63) agencés pour appliquer auxdites poches les pressions définies par lesdites première et seconde séquences fournies par le module de commande, de manière à gérer la circulation du fluide de culture dans ledit puits.

2. Dispositif selon la revendication 1, caractérisé en ce que lesdits premier (2) et second (25) réservoirs comprennent chacun une partie supérieure (3,26) et une partie inférieure (4,28) reliées entre elles par une partie intermédiaire étroite (5,30), chaque partie supérieure et inférieure des premier et second réservoirs logeant une poche souple, lesdites poches souples supérieure (6,27) et inférieure (7,29) communiquant entre elles via la partie intermédiaire (5,30), et lesdits moyens de liaison (20,24) communiquant avec les poches inférieures (6,29).

3. Dispositif selon la revendications 2, caractérisé en ce que ledit module de commande (50,150) est propre à fournir une première séquence de pressions externes supérieures pour la poche supérieure (6) du premier réservoir (2), une première séquence de pressions externes inférieures pour la poche inférieure (7) du premier réservoir (2), une seconde séquence de pressions externes inférieures pour ladite poche inférieure (29) du second réservoir (25) et une seconde séquence de pressions externes supérieures pour la poche supérieure (27) du second réservoir (25), et en ce que lesdits moyens de pressurisation (38,40-49,51-56,61-63) sont agencés pour appliquer auxdites poches supérieure (6) et inférieure (7) du premier réservoir (2) les pressions desdites premières séquences de pressions supérieures et inférieures, respectivement, et auxdites poches inférieure (29) et supérieure (27) du second réservoir (25) les pressions desdites secondes séquences de pressions inférieures et supérieures, respectivement, de manière à gérer la circulation du fluide de culture dans ledit puits.

4. Dispositif selon l'une des revendications 2 et 3, caractérisé en ce que lesdits niveaux supérieurs desdites poches souples supérieures (6,27) sont sensiblement identiques et lesdits niveaux inférieurs desdites poches souples inférieures (7,29) sont sensiblement identiques.

5. Dispositif selon l'une des revendications 1 à 4, caractérisé en ce que lesdites poches (6,7,27,29) sont perméables aux gaz, au moins dans le sens allant de l'extérieur vers l'intérieur.

6. Dispositif selon l'une des revendications 2 à 5, caractérisé en ce que lesdites poches supérieure (6) et inférieure (7) du premier réservoir (2), respectivement second réservoir (25), communiquent par un conduit (8,31) logé dans la partie intermédiaire (5,30).

7. Dispositif selon la revendication 6, caractérisé en ce que l'un au moins des premier (2) et second (25) réservoirs comporte un orifice (15) adapté propre à permettre l'introduction dudit fluide de culture dans le conduit (8,31) de communication entre poches.

8. Dispositif selon l'une dés revendications 2 à 7, caractérisé en ce que l'une au moins des parties supérieures (6,29) des réservoirs comporte une ouverture supérieure (10) agencée pour permettre le raccordement de la poche supérieure (6,29) qu'elle loge à un dispositif d'alimentation et/ou d'extraction de fluide de culture.

9. Dispositif selon la revendication 8, caractérisé en ce qu'il comprend des premiers moyens de contrôle d'accès (11,33), pilotés par le module de commande (50,150) et propres à contrôler l'accès à ladite poche supérieure (6,27) via ladite ouverture supérieure (10).

10. Dispositif selon l'une des revendications 2 à 9, caractérisé en ce que chaque partie inférieure (4,28) de réservoir comporte une ouverture inférieure (17) propre à permettre le raccordement desdits moyens de liaison (20,24) à la poche inférieure (7,29) qu'elle loge, et en ce qu'il comprend des seconds moyens de contrôle d'accès (13,35), pilotés par le module de commande (50,150) et propres à contrôler l'accès auxdites poches inférieures via lesdites ouvertures inférieures (17).

11. Dispositif selon l'une des revendications 2 à 10, caractérisé en ce que les parties supérieure (3,26) et inférieure (4,28) des premier (2) et second (25) réservoirs comportent chacune une entrée étanche (59), et en ce que lesdits moyens de pressurisation comprennent un premier circuit de pressurisation (40-43,46,47,56,58) propre à alimenter en fluide haute pression, sous le contrôle de vannes supérieures (47,147) et inférieures (46,146) pilotées par le module de commande (50), chaque partie supérieure (3,26) et inférieure (4,28) des premier et second réservoirs, via lesdites entrées étanches (59,17).

12. Dispositif selon l'une des revendications 2 à 10, caractérisé en ce que les parties supérieure (3,26) et inférieure (4,28) des premier (2) et second (25) réservoirs comportent chacune une entrée étanche (59), et en ce que lesdits moyens de pressurisation comprennent un premier circuit de pressurisation (40) comportant une pompe à fluide (41) et propre à alimenter en fluide haute pression, sous le contrôle de vannes supérieure (47) et inférieure (56) pilotées par le module de commande (50), chaque partie supérieure et inférieure du premier réservoir, via lesdites entrées étanches, ainsi qu'un second circuit de pressurisation (140) comportant une pompe à fluide (141) et propre à alimenter en fluide haute pression, sous le contrôle de vannes supérieure (147) et inférieure (156) pilotées par le module de commande (150), chaque partie supérieure et inférieure du second réservoir, via lesdites entrées étanches.

13. Dispositif selon l'une des revendications 11 et 12 en combinaison avec l'une des revendications 9 et 10, caractérisé en ce que lesdits premiers (11,33) et seconds (13,35) moyens de contrôle d'accès comprennent un conduit (64) muni d'une zone amincie (66) flexible logée dans une cavité (60) et en ce que chaque circuit de pressurisation comprend deux autres vannes (48,49 ;148,149) propres à alimenter en fluide haute pression les logements (60) desdits premier et seconds moyens de contrôle d'accès sur ordre dudit module de commande, pour agir sur ladite zone amincie (66) en vue de faire varier l'état du moyen de contrôle d'accès associé.

14. Dispositif selon l'une des revendications 11 et 13, caractérisé en ce que lesdits moyens de pressurisation comprennent un autre circuit de pressurisation raccordé auxdites vannes supérieures et inférieures, et propre à alimenter en fluide basse pression, sous le contrôle du module de commande, chaque partie supérieure et inférieure des premier et second réservoirs.

15. Dispositif selon l'une des revendications 12 et 13, caractérisé en ce que lesdits moyens de pressurisation comprennent un troisième circuit de pressurisation (51-55) raccordé auxdites vannes supérieure (47) et inférieure (46), et propre à alimenter en fluide basse pression, sous le contrôle du module de commande (50), chaque partie supérieure et inférieure du premier réservoir (2), ainsi qu'un quatrième circuit de pressurisation raccordé auxdites vannes supérieure (147) et inférieure (146) et propre à alimenter en fluide basse pression, sous le contrôle du module de commande, chaque partie supérieure et inférieure du second réservoir (25).

16. Dispositif selon la revendication 15 en combinaison avec la revendication 13, caractérisé en ce que chaque troisième et quatrième circuit de pressurisation est raccordé auxdites autres vannes (61,62 ;161,162) de manière à coopérer avec lesdits premier (11 ;33) et seconds (13 ;35) moyens de contrôle d'accès.

17. Dispositif selon l'une des revendications 2 à 16, caractérisé en ce qu'il comprend au moins deux puits (18-i) placés en série et communiquant entre eux par lesdits moyens de liaison (23), l'un des puits (18-1) étant raccordé audit premier réservoir (2) et l'autre puits (18-3) étant raccordé audit second réservoir (25).

18. Dispositif selon la revendication 17, caractérisé en ce qu'il comprend un troisième puits (18-3) placé en série entre les deux autres puits et communiquant avec eux par lesdits moyens de liaison (23).

19. Dispositif selon l'une des revendications 2 à 18, caractérisé en ce que lesdits moyens de liaison (20,23,24) sont constitués de conduits dont une extrémité au moins débouche en une chambre (22-i) de culture choisie de puits.

20. Dispositif selon l'une des revendications 17 à 19, caractérisé en ce que lesdits puits (18-i) sont des unités indépendantes assemblées les unes aux autres.

21. Dispositif selon l'une des revendications 17 à 19, caractérisé en ce que lesdits puits (18-i) sont agencés sur une plaque (66).

22. Dispositif selon l'une des revendications 1 à 21, caractérisé en ce que chaque chambre de culture (22-i) comporte au moins une paroi transparente (76) agencée pour permettre l'observation des cellules ou tissus en cours de culture.

23. Dispositif selon l'une des revendications 1 à 22, caractérisé en ce que certains au moins des puits sont agencés de manière à définir deux chambres de culture superposées et séparées par un filtre.

24. Dispositif selon l'une des revendications 1 à 23, caractérisé en ce que le module de contrôle (50 ;150) comprend une mémoire de type réinscriptible propre à stocker lesdites séquences de pression.

25. Installation de culture de cellules et tissus, caractérisée en ce qu'elle comprend au moins deux dispositifs (1-i) selon l'une des revendications 1 à 24, placés en parallèle, et une unité de commande propre à piloter conjointement les unités de commande (50-i,150-i) desdits dispositifs.

26. Installation selon la revendication 25, caractérisée en ce que certains puits d'un dispositif communiquent avec au moins un autre puits d'un autre dispositif, via lesdits moyens de liaison.

27. Procédé de culture de cellules et tissus, du type comprenant une première étape dans laquelle on prévoit un dispositif de culture comportant au moins un puits de culture propre à recevoir des cellules ou des tissus à cultiver, de premier et second réservoirs couplés au puits, l'un au moins de ces deux réservoirs étant agencé pour recevoir un fluide de culture, caractérisé en ce que dans la première étape on loge dans chaque réservoir au moins une poche souple, l'une au moins de ces poches étant propre à recevoir le fluide de culture, et en ce qu'il comprend une seconde étape dans laquelle on applique auxdites poches des pressions définies par des première et seconde séquences de pressions choisies, pour gérer la circulation du fluide de culture dans ledit puits.

28. Procédé selon la revendication 27, caractérisé en ce que dans la première étape on prévoit dans chaque réservoir une partie supérieure et une partie inférieure reliées entre elles par une partie intermédiaire étroite et logeant chacune une poche souple, lesdites poches supérieure et inférieure communiquant entre elles via la partie intermédiaire et lesdites poches inférieures étant couplées au puits, et en ce que dans la seconde étape on applique sur lesdites poches supérieure et inférieure du premier réservoir des pressions définies par des premières séquences de pressions supérieures et inférieures choisies, respectivement, et sur lesdites poches inférieure et supérieure du second réservoir des pressions définies par des secondes séquences de pressions inférieures et supérieures choisies, de manière à gérer la circulation du fluide de culture dans ledit puits.

29. Procédé selon l'une des revendications 27 et 28, caractérisé en ce que dans la seconde étape la première séquence appliquée à chaque poche du premier réservoir consiste en une alternance de premières périodes de haute pression et de secondes périodes de basse pression, et la seconde séquence appliquée à chaque poche du second réservoir consiste en une alternance de premières périodes de basse pression et de secondes périodes de haute pression.

30. Procédé selon la revendication 28, caractérisé en ce que dans la seconde étape la première séquence appliquée à la poche supérieure du premier réservoir consiste en une alternance de premières périodes de basse pression et de secondes périodes de haute pression, et la seconde séquence appliquée à la poche supérieure du second réservoir consiste en une alternance de premières périodes de haute pression et de secondes périodes de basse pression, et en ce que les première et seconde séquences appliquées respectivement aux poches inférieures des premier et second réservoir consistent en une succession de périodes de haute pression.

31. Procédé selon la revendication 28, caractérisé en ce que dans la seconde étape la première séquence appliquée à la poche inférieure du premier réservoir consiste en une alternance de premières périodes de basse pression et de secondes périodes de haute pression, et la seconde séquence appliquée à la poche inférieure du second réservoir consiste en une alternance de premières périodes de haute pression et de secondes périodes de basse pression, et en ce que les première et seconde séquences appliquées respectivement aux poches supérieures des premier et second réservoir consistent en une succession de périodes de haute pression.

32. Procédé selon l'une des revendications 29 à 31, caractérisé en ce que dans la seconde étape les durées des premières périodes des premières et secondes séquences sont sensiblement identiques, et les durées des secondes périodes des premières et secondes séquences sont sensiblement identiques.

33. Procédé selon la revendication 29, caractérisé en ce que dans la seconde étape les durées des premières périodes des premières et secondes séquences sont sensiblement identiques, et les durées des secondes périodes des premières et secondes séquences sont sensiblement identiques.

34. Procédé selon l'une des revendications 28 à 32, caractérisé en ce que dans la seconde étape :
* la première séquence supérieure appliquée à la poche supérieure du premier réservoir consiste en une suite répétée un nombre de fois choisi de première, seconde, troisième et quatrième périodes de basse pression ;
* la première séquence inférieure appliquée à la poche inférieure du premier réservoir consiste en une suite répétée ledit nombre de fois choisi d'une première période de haute pression, d'une seconde période de haute pression, d'une troisième période de haute pression et d'une quatrième période de basse pression ;
* la seconde séquence inférieure appliquée à la poche inférieure du second réservoir consiste en une suite répétée ledit nombre de fois choisi d'une première période de haute pression, d'une seconde période de basse pression, d'une troisième période de haute pression et d'une quatrième période de haute pression ; et
* la seconde séquence supérieure appliquée à la poche supérieure du second réservoir consiste en une suite répétée ledit nombre de fois choisi d'une première, seconde, troisième et quatrième périodes de basse pression.

35. Procédé selon la revendication 34, caractérisé en ce que dans la seconde étape les durées des premières périodes des premières et secondes séquences supérieure et inférieure sont sensiblement identiques, les durées des secondes périodes des premières et secondes séquences supérieure et inférieure sont sensiblement identiques, les durées des troisièmes périodes des premières et secondes séquences supérieure et inférieure sont sensiblement identiques, et les durées des quatrièmes périodes des premières et secondes séquences supérieure et inférieure sont sensiblement identiques.
